# EUROPEAN PATENT APPLICATION

(11) **EP 0 619 371 A1**
(43) Date of publication of application: **12.10.1994**
(21) Application number: 94105165.8
(22) Date of filing: 31.03.1994
(51) Int. Cl.: C12N 15/19, C07K 13/00, C12P 21/02, A61K 37/02, C12P 21/08, C12N 1/21

(54) **Polypeptide derived from a glioblastoma cell line, its use and DNAs encoding it**

(30) Priority: 06.04.1993 JP 79365/93
(71) Applicant: ONO PHARMACEUTICAL CO., LTD., Osaka-shi Osaka (JP)
(72) Inventor: Naito, Takayuki, Ibaraki-shi, Osaka 567 (JP); Hirano, Atsushi, c/o Minase Research Institute, Shimamotocho, Mishima-gun, Osaka 618 (JP); Miyamoto, Tsumoru, Shiga-gun, Shiga 520-05 (JP)
(74) Representative: Henkel, Feiler, Hänzel & Partner

(57) **Abstract**

The present invention is related to novel polypeptide consisting of 253 amino acids which is produced in a human glioblastoma cell line, a method of producing it, DNA encoding the said polypeptide, a fragment capable of selectively hybridizing to the DNA sequence, a replication or expression vector comprising the DNA and a host cell transformed with the replication or expression vector. The polypeptide of the present invention may be useful for the prevention of or in the treatment of aplasia or abnormal proliferation, of glias, neurons or hematopoietic cells, depression or enhancement of immunological or neurological activity, or tumors or inflammatory diseases.

## Description

### Field of the Invention

The present invention is related to a novel peptide produced by a certain cell line and DNAs encoding it.

Cells which constitute a brain may be largely grouped into neuron and glia. Neuron plays a main role in the transmission and treatment of information in a brain.

On the other hand, glia supports the action of neuron. Concretely, it is considered that glia is associated with protection and support of neuron, formation of myelin sheath, formation of blood brain barrier, delivery of nutrition to neuron and metabolism of neurotransmitter, and further is associated with the control of proliferation and differentiation of neuron when the brain is growing, etc. Glia contains various kinds of cells. There are astrocytes, oligo-dendrocytes, microglias etc. in central nervous system, and Schwann cells, mantle cells etc. in peripheric nervous system. Ependymal cells existing in cerebral ventricular endothelium belongs to glia.

### Related Arts

Recently, many humoral factors relating to differentiation, proliferation and growth, of neurons and glia, have been found, and most of them is generated from glia or tumorous glia (referred as glia etc. as a whole hereafter).

For example, nerve growth factor (NGF, m.w. ca. 13.5 kd (human)), brain-derived neurotrophic factor (BDNF, m.w. ca. 13.5 kd (human)), neuroblastoma growth inhibitory factor (NGIF, m.w. ca. 5 kd (human)) and glia-derived neurotrophic factor (m.w. ca. 500 kd and ca. 110 kd (both rats)), which all are known as factors acting on neuron, are generated from astrocytes. Further, glia-derived glial growth inhibitory factor (GdGGIF, m.w. more than 100 kd (rats)) and platelet-derived growth factor (PDGF, m.w. ca. 32 kd (human)), both acting on glia, are also generated from astrocytes. Furthermore, glia-derived neurotrophic factor (GdNTF, m.w. ca. 43 kd (rats)) exhibiting a neurotrophic activity and a proliferating activity on glia, glia maturation factor (GMF, m.w. ca. 16.5 kd (bovine)) exhibiting a neurotrophic activity and a stimulatory activity on proliferation and differentiation of glioblastoma, cilially neurotrophic factor (CNTF, m.w. ca. 23 kd (rabbits and rats)) exhibiting an activity of life maintenance of ganglion cells, an inhibitory activity of proliferation of sympathetic ganglion cells, a stimulatory activity of differentiation of sympathetic ganglion cells and astrocytes, and fibroblast growth factor (FGF, m.w. ca. 16 to 17 kd (human)) exhibiting stimulatory activity of proliferation of neurons and astrocytes, all are generated from astrocytes. Schwann cell-derived neuron growth factor (SchNTF, m.w. more than 8 kd (rats)) acting on neuron is generated from Schwann cells (see PROTEIN, NUCLEIC ACID AND ENZYME, vol. 36 (No. 7), pp. 260, 1991 in detail). It has become known that interleukines (IL-1, IL-6 etc.) which immunocytes is considered to mainly secret, is also produced from glia.

Amino acid sequences of most of these factors have been revealed. At present they have been treated as a subject of not only pure research but also research and development for the purpose of applying to pharmaceuticals.

### Purpose of the Invention

As mentioned above, it is understood that glia etc. generate many humoral factors relating to differentiation, proliferation and growth, of neuron and glia, and relating to immunity. These facts show the possibility that some factors other than the above factors are generated from glia.

Present inventors have directed their attention to this point and energetic research has been carried out in order to find novel factors (polypeptides) which glia etc. generate.

Until now, when a man skilled in the art intends to obtain a particular polypeptide or a DNA encoding it, he generally utilizes methods by confirming an intended biological activity in a tissue or in a cell medium, isolating and purifying the polypeptide and then cloning a gene or methods by "expression-cloning" with the guidance of the biological activity.

However, physiologically active polypeptides in living body have often many kinds of activities. Therefore, it is increasing that after a gene is cloned, the gene is found to be identical to that encoding a polypeptide already known. Generally glia generates only a very slight amount of a factor and it makes difficult to isolate and to purify the factor and to confirm its biological activity.

On the other hand, preparation technique of cDNA and sequencing technique have been rapidly developed, and it has been able to sequence a large quantity of cDNA. Further, methods of "Reverse Genetics" of characterizing the function of a gene from the sequence of the gene, have been greatly developed.

The present inventors attempted to find novel polypeptides by using these methods. That is, a series of methods was carried out by isolating mRNA from glia etc., obtaining cDNA by using mRNA thus obtained, deciding its nucleotide sequence and deducing its amino acid sequence. In this manner, the present inventors have succeeded to find a quite novel polypeptide and DNA encoding it, to complete the present invention.

There was no polypeptide having amino acid sequence which is identical to or has high homology to that of the polypeptide of the present invention, when amino acid sequences of the polypeptide was compared by a computer to all known sequences in data base of Swiss Prot (Swiss Prot Release 2.0). Furthermore, there was no nucleotide sequence which is identical to or has high homology to that encoding the polypeptide of the present invention, when the DNA sequence was compared by a computer to all known sequences in data base of GenBank (GenBank Release 70.0). And there was two unstabilizing sequence motif ATTTA, which was identified in many cytokaines in its 3'-non coding region. So, this polypeptide was considered that they had cytokine-like activity. Therefore, the polypeptide of the present invention is considered to be quite novel.

### Constitution of the Invention

The present invention is concerned with a polypeptide having the amino acid shown in SEQ ID. No. 1, in substantially purified form, a homologue thereof or a fragment of the sequence or homologue of a fragment, and DNA encoding such a polypeptide. More particularly, the present invention is related to DNA having the nucleotide sequence shown in SEQ ID No. 2 or 3, and DNA having a fragment which is selectively hybridizing to nucleotide sequence shown in SEQ ID No. 2 or 3.

The present invention is related to:
(1) a polypeptide having an amino acid sequence shown in SEQ ID NO. 1,
(2) a DNA encoding the polypeptide described above (1),
(3) a DNA having a nucleotide sequence shown in SEQ ID NO. 2, and
(4) a DNA having a nucleotide sequence shown in SEQ ID NO. 3.

A polypeptide of Seq. ID No. 1 in substantially purified form will generally comprise the polypeptide in a preparation in which more than 90%, e.g. 95%, 98% or 99% of the polypeptide in the preparation is that of the Seq. ID No. 1.

A polypeptide homologue of the Seq. ID No. 1 will be generally at least 70%, preferably at least 80 or 90% and more preferably at least 95% homologous to the polypeptide of Seq. ID No. 1 over a region of at least 20, preferably at least 30, for instance 40, 60 or 100 more contiguous amino acids. Such polypeptide homologues will be referred to below as a polypeptide according to the invention.

Generally, fragments of Seq. ID No. 1 or its homologues will be at least 10, preferably at least 15, for example 20, 25, 30, 40, 50 or 60 amino acids in length, and are also encompassed by the term "a polypeptide according to the invention" as used herein.

A DNA capable of selectively hybridizing to the DNA of Seq. ID No. 2 or 3 will be generally at least 70%, preferably at least 80 or 90% and more preferably at least 95% homologous to the DNA of Seq. ID No. 2 or 3 over a region of at least 20, preferably at least 30, for instance 40, 60 or 100 or more contiguous nucleotides. Such DNA will be encompassed by the term "DNA according to the invention".

Fragments of the DNA of Seq. ID No. 2 or 3 will be at least 15, preferably at least 20, for example 25, 30 or 40 nucleotides in length, and are also encompassed by the term "DNA according to the invention" as used herein.

A further embodiment of the invention provides replication and expression vectors comprising DNA according to the invention. The vectors may be, for example, plasmid, virus or phage vectors provided with an origin of replication, optionally a promoter for the expression of the said DNA and optionally a regulator of the promoter. The vector may contain one or more selectable marker genes, for example a anpicillin resistance gene. The vector may be used in vitro, for example of the production of RNA corresponding to the DNA, or used to transfect or transform a host cell.

A further embodiment of the invention provides host cells transformed or transfected with the vectors for the replication and expression of DNA according to the invention, including the DNA SEQ. ID No. 2 or 3 or the open reading frame thereof. The cells will be chosen to be compatible with the vector and may for example be bacterial, yeast, insect or mammalian.

A further embodiment of the invention provides a method of producing a polypeptide which comprises culturing host cells of the present invention under conditions effective to express a polypeptide of the invention. Preferably, in addition, such a method is carried out under conditions in which the polypeptide of the invention is expressed and then produced from the host cells.

DNA according to the invention may also be inserted into the vectors described above in an antisense orientation in order to proved for the production of antisense RNA. Antisense RNA may also be produced by synthetic means. Such antisense RNA may be used in a method of controlling the levels of a polypeptide of the invention in a cell.

The invention also provides monoclonal or polyclonal antibodies to a polypeptide according to the invention. The invention further provides a process for the production of monoclonal or polyclonal antibodies to the polypeptides of the invention. Monoclonal antibodies may be prepared by conventional hybridoma technology using a polypeptide of the invention or a fragment thereof, as an immunogen. Polyclonal antibodies may also be prepared by conventional means which comprise inoculating a host animal, for example a rat or a rabbit, with a polypeptide of the invention and recovering immune serum.

The present invention also provides pharmaceutical compositions containing a polypeptide of the invention, or an antibody thereof, in association with a pharmaceutically acceptable diluent and/or carrier.

The polypeptide of the present invention includes that which a part of their amino acid sequence is lacking (e.g., a polypeptide comprised of the only essential sequence for revealing a biological activity in an amino acid sequence shown in SEQ ID No.1), that which a part of their amino acid sequence is replaced by other amino acids (e.g., those replaced by an amino acid having a similar property) and that which other amino acids are added or inserted into a part of their amino acid sequence, as well as those having the amino acid sequence shown in SEQ ID NO. 1.

As known well, there are one to six kinds of codon as that encoding one amino acid (for example, one kind of codon for Methionine (Met), and six kinds of codon for leucine (Leu) are known). Accordingly, the nucleotide sequence of DNA can be changed in order to encode the polypeptide having the same amino acid sequence.

The DNA of the present invention, specified in (2) includes a group of every nucleotide sequences encoding polypeptides (1) shown in SEQ ID NO. 1 . There is a probability of improving a yield of production of a polypeptide by changing a nucleotide sequence.

The DNA specified in (3) is the embodiment of DNA shown in (2), and is sequence in the natural form.

The DNA shown in (4) indicates the sequence of the DNA specified in (3) with a non-translational region.

The DNA having a nucleotide sequence shown in SEQ ID NO. 3 may be prepared according to the following methods, that is:
(i) by isolating mRNA from a cell line which products the polypeptide of the present invention (e.g., human glioblastoma cell line),
(ii) by preparing first strand (single stranded DNA) from mRNA thus obtained, followed by preparing second strand (double stranded DNA) (synthesis of cDNA),
(iii) by inserting cDNA thus obtained into a proper plasmid vector,
(iv) by transforming host cells with the recombinant DNA thus obtained (preparation of cDNA library),
(v) by random-cloning on a large scale from cDNA library thus obtained, followed by sequencing about 300 bases from 5' end of each clone, and
(vi) by sequencing complete length of a clone which has a novel base sequence.

Explained in detail, step (i) may be carried out in accordance with the method of Okayama, H. et al. (described in Methods in Enzymology, vol. 154, p 3, 1987) after a human glioblastoma cell line is stimulated by a proper stimulant (e.g., IL-1 etc.). Examples of the cells which product the polypeptide of the present invention is preferably human glioblastoma cell line T98G (ATCC strain No., CRL-1690).

Steps (ii), (iii) and (iv) are a series of steps for preparing cDNA library, and may be carried out in accordance with the method of Gubler & Hoffman (Gene, vol. 25, pp. 263, 1983) with a slight modification. As examples of the plasmid vector used in the step (iii), many vectors functioning in an E. coli strain (e.g., pBR 322) and in a Bacillus subtilis (e.g., pUB 110) are known, and pVfCS-1 (described in detail in the following Example) prepared from pGEM-3Zf(+) (3,199 bp, manufactured by Promega Corp.) which functions in an E. coli, may be preferably used.

The cloning in the step (v) may be carried out by methods known per se and the sequencing may be carried out in accordance with the method of Maxam-Gilbert or the dideoxy termination method.

The step (vi) may be carried out in accordance with the method described in Molecular Cloning (written by Sambrook, J., Fritsch, E. F. and Maniatis, T., published. by Cold Spring Harbor Laboratory Press in 1989).

As the following step, it is necessary to examine whether or not the DNA thus obtained codes right a secretory protein. The examination requires:
(I) the conversion of the DNA sequence into the amino acid sequence in a possible frame,
(II) the confirmation that the DNA thus obtained covers complete or almost complete length of intact mRNA. These confirmation may be carried out after the step (vi) hereinbefore described, and effectively between the step (v) and the step (vi).

The Step (II) may be carried out by Northern analysis.

Once the nucleotide sequences shown in SEQ ID NOs. 2 and 3 are determined, DNA of the present invention may be obtained by chemical synthesis, by PCR method or by hybridization making use of a fragment of DNA of the present invention, as a probe. Furthermore, DNA of the present invention may be obtained in a desired amount by transforming with a vector DNA inserted a DNA of the present invention into a proper host, followed by culturing the transformant.

The polypeptides of the present invention (shown in SEQ ID NO. 1) may be prepared by:
(1) isolating and purifying from an organism or a cultured cell,
(2) chemically synthesizing, or
(3) using a skill of biotechnology,
preferably, by the method described in (3).

Examples of expression system when preparing a polypeptide by using a skill of biotechnology, is, for example, the expression system of bacteria, yeast, insect cell and mammalian cell.

For example, the expression in E. coli may be carried out by adding the initiation codon (ATG) to 5' end of a DNA encoding a nucleotide sequence shown in SEQ ID NO. 3, connecting the DNA thus obtained to the downstream of a proper promoter (e.g., trp promoter, lac promoter, λPL promoter, T7 promoter etc.), and then inserting it into a vector (e.g., pBR322, pUC18, pUC19 etc.) which functions in an E. coli strain to prepare an expression vector.

Then, an E. coli strain (e.g., E. coli DH1 strain, E. coli JM109 strain, E. coli HB101 strain, etc.) which is transformed with the expression vector thus obtained may be cultured in a proper medium to obtain the desired polypeptide. When a signal peptide of bacteria (e.g., signal peptide of pel B) is utilized, the desired polypeptide may be also produced in periplasm. Furthermore, a fusion protein with other polypeptide may be also produced easily.

Furthermore, the expression in a mammalian cell may be carried out, for example, by inserting the DNA shown in SEQ ID NO. 3 into the downstream of a proper promoter (e.g., SV40 promoter, LTR promoter, metallothionein promoter etc.) in a proper vector (e.g., retrovirus vector, papilloma virus vector, vaccinia virus vector, SV40 vector, etc.) to obtain an expression vector, and transforming a proper mammalian cell (e.g., monkey COS-7 cell, Chinese hamster CHO cell, mouse L cell etc.) with the expression vector thus obtained, and then culturing the transformant in a proper medium to get a desired polypeptide in the culture medium. The polypeptide thus obtained may be isolated and purified by conventional biochemical methods.

### Effects of the Invention

The polypeptide of the present invention is synthesized in a human glioblastoma cell line, and therefore, may possess biological activities relating to differentiation, proliferation and growth, of glias or neurons, relating to the function of immunity and relating to proliferation and growth, of tumors or inflammatory diseases. The polypeptide of the present invention is useful for the prevention of or in the treatment of aplasia or abnormal proliferation, of glias, neurons or hematopoietic cells, depression or enhancement of immunological or neurological activity, or tumors etc.

Further, polyclonal or monoclonal antibody against the polypeptide of the present invention can be used in the determination of the amount of the said polypeptide in organism, and thereby, may be utilized for the purpose of investigating the relationship between the said polypeptide and diseases, or for the purpose of diagnosing diseases, and the like. Polyclonal and monoclonal antibody thereof may be prepared by conventional methods by using the said polypeptide or the fragment thereof as an antigen.

The DNA of the present invention may be utilized as an important and essential template in preparing the polypeptide of the present invention which is expected to possess various use or for diagnosis of and in the treatment of gene diseases (the treatment of gene defect disease and the treatment by inhibiting expression of the polypeptide by antisense DNA (RNA), and the like). Further, genomic DNA may be isolated by using the DNA of the present invention as a probe. Similarly, it is possible to isolate genes having high homology to the DNA of the present invention in human or those of other species.

### Application for Pharmaceuticals

For the purpose of the present invention of or in the treatment of aplasia or abnormal proliferation, of glia or neurons, depression or enhancement of immunological activity, or tumours etc., the polypeptide of the present invention may be normally administered systemically or partially, usually by oral or parenteral administration, preferably orally, intravenously or intraventricularly.

The doses to be administered are determined depending upon age, body weight, symptom, the desired therapeutic effect, the route of administration, and the duration of the treatment etc. In the human adult, the doses per person per dose are generally between 100 µg and 100 mg, by oral administration, up to several times per day, and between 10 µg and 100 mg, by parenteral administration up to several times per day.

As mentioned above, the doses to be used depend upon various conditions. Therefore, there are cases in which doses lower than or greater than the ranges specified above may be used.

Administration of the compounds of the present invention, may be as solid compositions, liquid compositions or other compositions for oral administration, as injections, liniments or suppositories etc. for parenteral administration.

Solid compositions for oral administration include compressed tablets, pills, capsules, dispersible powders, granules. Capsules include soft capsules and hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate, etc.). The compositions may also comprise, as is normal practice, additional substances other than inert diluents: e.g. lubricating agents (such as magnesium stearate etc.), disintegrating agents (such as cellulose calcium glycolate, etc.), stabilizing agents (such as human serum albumin, lactose etc.), and assisting agents for dissolving (such as arginine, asparaginic acid etc.).

The tablets or pills may, if desired, be coated with a film of gastric or enteric material (such as sugar, gelatin, hydroxypropyl cellulose or hydroxypropylmethyl cellulose phthalate, etc.), or be coated with more than two films. And further, coating may include containment within capsules of absorbable materials such as gelatin.

Liquid compositions for oral administration include pharmaceutically-acceptable emulsions, solutions, syrups and elixirs. In such compositions, one or more of the active compound(s) is or are contained in inert diluent(s) commonly used in the art (purified water, ethanol etc.). Besides inert diluents, such compositions may also comprise adjuvants (such as wetting agents, suspending agents, etc.), sweetening agents, flavouring agents, perfuming agents, and preserving agents.

Other compositions for oral administration included spray compositions which may be prepared by known methods and which comprise one or more of the active compound(s). Spray compositions may comprise additional substances other than inert diluents: e.g. stabilizing agents (sodium sulfite etc.), isotonic buffer (sodium chloride, sodium citrate, citric acid, etc.). For preparation of such spray compositions, for example, the method described in the United States Patent No. 2,868,691 or 3,095,355 (herein incorporated in their entireties by reference) may be used.

Injections for parenteral administration include sterile aqueous or non-aqueous solutions, suspensions and emulsions. In such compositions, one or more active compound(s) is or are admixed with at least one inert aqueous diluent(s) (distilled water for injection, physiological salt solution, etc.) or inert non-aqueous diluents(s)(propylene glycol, polyethylene glycol, olive oil, ethanol, POLYSOLBATE 80™ , etc.).

Injections may comprise additional other than inert diluents: e.g. preserving agents, wetting agents, emulsifying agents, dispersing agents, stabilizing agent (such as human serum albumin, lactose, etc.), and assisting agents such as assisting agents for dissolving (arginine, asparaginic acid, etc.).

They may be sterilized for example, by filtration through a bacteria-retaining filter, by incorporation of sterilizing agents in the compositions or by irradiation. They may also be manufactured in the form of sterile solid compositions, for example, by freeze-drying, and which can be dissolved in sterile water or some other sterile diluents for injection immediately before used.

Other compositions for parenteral administration include liquids for external use, and endermic liniments (ointment, etc.), suppositories for rectal administration and pessaries which comprise one or more of the active compound(s) and may be prepared by known methods.

### Examples

The following examples are illustrated, but not limit, the present invention.

### Example 1: Construction of vector for use in the preparation of cDNA library

A plasmid vector, pGEM-3Zf(+) (3,199 bp, available from Promega Corp.) was digested with HindIII, followed by Klenow treatment and circularized again. The necessary amount of this plasmid was recovered from culture of E. coli transformed with the plasmid. Next, an AatII-NdeI fragment was cut out from the plasmid, and the resulting linear plasmid fragment was smooth-ended using T4 polymerase. After ligating the thus treated termini with a HindIII linker, the resulting fragment was digested with HindIII, circularized again and then transformed into an E. coli strain to recover plasmid.

Thereafter, a SacI-PstI portion in the polylinker of the plasmid was replaced by a synthetic polylinker shown below:

NotI
SmaI
SalI BglII KpnI SpeI
5' CGTCGACAGATCTGGTACCCGGGCGGCCGCACTAGTCTGCA 3' (SEQ ID NO.5)
3' TCGAGCAGCTGTCTAGACCATGGGCCCGCCGGCGTGATCAG 5' (SEQ ID NO.6)

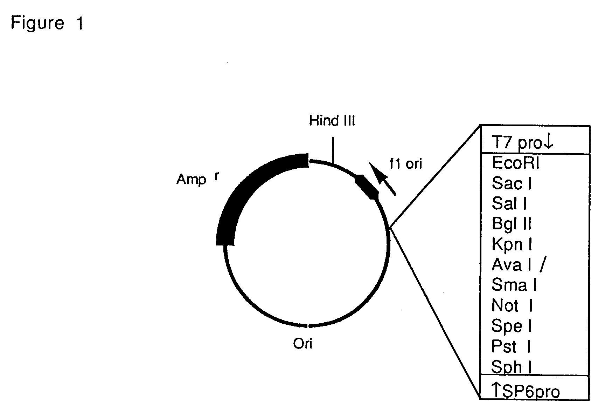

The plasmid vector thus constructed (see Fig. 1) was named pVfCS-1.

The pVfCS-1 has the following characteristic properties as a multi-purpose plasmid vector.
1. Okayama-Berg method and Gubler-Hoffman method can be applied.
2. Plasmid yield per cultured cells is high.
3. Single-stranded DNA can be prepared.
4. A cDNA insert can be cut out easily.
5. Preparation of a deletion mutant for sequencing use can be made easily.
6. In vitro transcription can be made.

### Example 2: Isolation and purification of mRNA

A total of 3 x 10⁷ cells of a human glioblastoma cell line T98G (ATCC strain No., CRL-1690) were stimulated for 4 hours with 100 units/ml of human IL-1β, subsequently isolating mRNA in accordance with the method of Okayama, H. et al. (Methods in Enzymology, vol. 154, pp. 3, 1987) as described below.

That is, the stimulated cells were solubilized with a 5.5 M GTC solution (5.5 M guanidine thiocyanate, 25 mM sodium citrate, 0.5% sodium lauryl sarcosine), and the resulting cell lysate was laid on a cushion of a cesium trifluoroacetate (CsTFA) solution having a density of 1.51 and centrifuged (120,000 x g, 20 hours) to recover 1.26 mg of total RNA in the resulting pellet. Thereafter, the RNA sample was passed twice through an oligo(dT)-cellulose column to purify and recover 46 µg of poly(A)+RNA.

### Example 3: Preparation of cDNA library

A cDNA library was prepared in accordance with the method of Gubler & Hoffman (Gene, vol. 25, pp. 263, 1983) with a slight modification.

A first strand was synthesized from the poly(A)⁺ RNA (5 µg) prepared in Example 2, using a reverse transcriptase and an oligo(dT) primer having a NotI site. After synthesizing a second strand and carrying out SalI adapter ligation and Notl digestion, the adapter and the primer were removed by gel filtration column chromatography using a column packed with Sephacryl S-500HR (available from Pharmacia), thereby recovering a fraction containing 820 ng of cDNA.

The above cDNA synthesizing step was effected making use of a kit (Super Script System, available from BRL).

Separately from this, a vector was prepared by subjecting the pVfCS-1 obtained in Example 1 to complete digestion with NotI, digesting the product further with SalI, subjecting the resulting digest to 0.8% agarose gel electrophoresis to cut out a band of interest and then purifying the vector of interest making use of a kit for glass powder method use (GENECLEAN II, available from BIO 101).

After subjecting the thus prepared cDNA and vector to ligation, the resulting product was transfected into DH5 competent cells which have been prepared in accordance with the method of Inoue, H. et al. (Gene, vol. 96, pp. 23, 1990). As the results, a cDNA library containing 6 x 10⁵ independent clones with an average length of 1.5 kb was obtained.

### Example 4: Cloning and Sequencing

Using the cDNA library prepared in Example 3, clones were plated on LB-broth agar containing ampicillin with a density of 300 colonies/dish having a diameter of 10 cm. Cloning was carried out by picking up the colonies at random. Each of the colonies was cultured overnight in 3 ml of LB-broth. A 200 µl portion of the resulting culture was mixed with dimethylsulfoxide (DMSO, final concentration of 7%) and stored at -80°C, and the remaining portion of the culture was used for the isolation of plasmid. The purification of plasmid was carried out by the usual way.

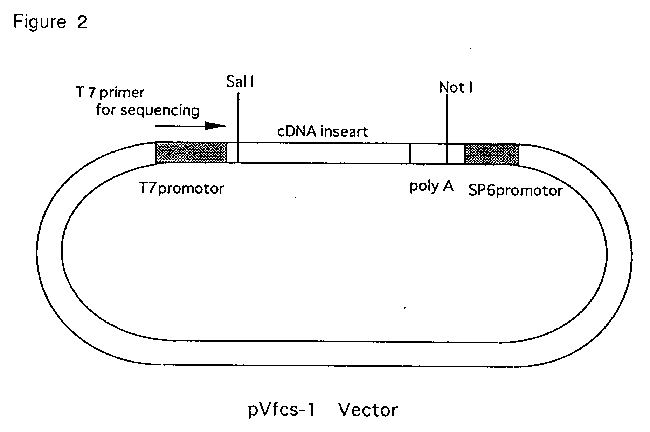

Since the plasmid has a structure shown in Fig. 2, its nucleotide sequence can be read from the 5' end of the cloned cDNA when sequencing is carried out using T7 primer.

DNA sequencing was carried out in accordance with a cycle sequence method based on the dideoxy termination method of Sanger, F. et al., using a fluorescence dye terminator of ABI (Applied Biosystems Inc.). Reading of the sequence was carried out using a DNA sequencer of ABI (Model 373A). In this way, a nucleotide sequence having a length of about 300 bases from 5' end of each cDNA was obtained.

### Example 5: Analysis of partial sequence data

Using the FASTA program of Lipman, D. J. and Pearson, W. R., the nucleotide sequence obtained in Example 4 was searched for its homology with every nucleotide sequence contained in known data bases (GenBank and EMBL). As the results, a clone having an unknown sequence was identified. The unknown nucleotide sequence was converted into amino acid sequences in possible three frames.

It is possible however that the cloned cDNA does not cover complete length of mRNA. If it does not cover the complete length, it is less possible that the clone contains the N-terminal amino acid sequence moiety.

In consequence, Northern analysis was carried out in order to determine if the clone TG0847 has complete length or not. That is, poly(A)+RNA which has been extracted and purified from a glioblastoma cell line was subjected to electrophoresis and then blotting on nylon membrane.

When hybridization was carried out using a TG0847 cDNA insert as a probe, a single band was found at a position corresponding to about 1900 bp. Since the TG0847 cDNA insert had a size of about 1600 bp, it was confirmed that the TG0847 clone was a almost complete length cDNA.

### Example 6: Determination of complete sequence of the cDNA and open reading frame

The complete length cDNA sequence was determined by means of random sequencing in accordance with the method described in Molecular Cloning (Sambrook, J., Fritsch, E. F. and Maniatis, T., Cold Spring Harbor Laboratory Press, 1989).

Plasmid was recovered from the TG0847 clone to isolate and purify a cDNA insert. The insert thus purified was subjected to ligation and fragmentation, followed by smooth-ending of both termini of the resulting DNA fragment with T4 polymerase, thereby recovering a DNA fragment of about 400 bp in length by agarose gel electrophoresis. The thus obtained DNA fragment was cloned into a Smal site of a plasmid vector BLUESCRIPT II (available from Stratagene) and then transfected into an E. coli strain. A total of 20 colonies were picked up at random to prepare 20 corresponding plasmid DNA samples (every of them contained a TG0847 cDNA fragment as an insert) which were subsequently subjected to DNA sequencing.

DNA sequencing and sequence reading were carried out in the same manner as the procedure described in Example 4. Sequence data of the TG0847 cDNA fragment were arranged into a continued sequence making use of a DNA sequence connection program of DNASIS, thereby obtaining a base sequence shown in SEQ ID NO. 3. An open reading frame was determined based on the complete length cDNA sequence data to deduce corresponding amino acid sequence, with the results shown in SEQ ID NO. 1.

Complete nucleotide sequence of the TG0847 cDNA and primary amino acid sequence of the TG0847 protein encoded by the nucleotide sequence are shown in SEQ ID NO. 4.

SEQUENCE LISTING

(1) GENERAL INFORMATION:

(i) APPLICANT:
(A) NAME: Ono Pharmaceutical Co., Ltd.
(B) STREET: 1-5, Doshomachi 2-chome
(C) CITY: Chuo-ku, Osaka-shi
(D) STATE: Osaka
(E) COUNTRY: Japan
(F) POSTAL CODE (ZIP): 541

(ii) TITLE OF INVENTION: Novel Polypeptide and DNAs Encoding It

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 253 amino acids
(B) TYPE: amino acid
(D) TOPOLOGY: linear

(ii) MOLECULE TYPE: protein

(xi) SEQUENCE DESCRIPTION: SEQ ID NO: 1:

Met Ala Ser Gly Ser Asn Trp Leu Ser Gly Val Asn Val Val Leu Val
1               5                   10                  15

Met Ala Tyr Gly Ser Leu Val Phe Val Leu Leu Phe Ile Phe Val Lys
            20                  25                  30

Arg Gln Ile Met Arg Phe Ala Met Lys Ser Arg Arg Gly Pro His Val
        35                  40                  45

Pro Val Gly His Asn Ala Pro Lys Asp Leu Lys Glu Glu Ile Asp Ile
    50                  55                  60

Arg Leu Ser Arg Val Gln Asp Ile Lys Tyr Glu Pro Gln Leu Leu Ala
65                  70                  75                  80

Asp Asp Asp Ala Arg Leu Leu Gln Leu Glu Thr Gln Gly Asn Gln Ser
                85                  90                  95

Cys Tyr Asn Tyr Leu Tyr Arg Met Lys Ala Leu Asp Ala Ile Arg Thr

            100                 105                 110

Ser Glu Ile Pro Phe His Ser Glu Gly Arg His Pro Arg Ser Leu Met
        115                 120                 125

Gly Lys Asn Phe Arg Ser Tyr Leu Leu Asp Leu Arg Asn Thr Ser Thr
    130                 135                 140

Pro Phe Lys Gly Val Arg Lys Ala Leu Ile Asp Thr Leu Leu Asp Gly
145                 150                 155                 160

Tyr Glu Thr Ala Arg Tyr Gly Thr Gly Val Phe Gly Gln Asn Glu Tyr
                165                 170                 175

Leu Arg Tyr Gln Glu Ala Leu Ser Glu Leu Ala Thr Ala Val Lys Ala
            180                 185                 190

Arg Ile Gly Ser Ser Gln Arg His His Gln Ser Ala Ala Lys Asp Leu
        195                 200                 205

Thr Gln Ser Pro Glu Val Ser Pro Thr Thr Ile Gln Val Thr Tyr Leu
    210                 215                 220

Pro Ser Ser Gln Lys Ser Lys Arg Ala Lys His Phe Leu Glu Leu Lys
225                 230                 235                 240

Ser Phe Lys Asp Asn Tyr Asn Thr Leu Glu Ser Thr Leu
                245                 250

(2) INFORMATION FOR SEQ ID NO: 2:

(i) SEQUENCE CHARACTERISTICS:
(A) LENGTH: 759 base pairs
(B) TYPE: nucleic acid
(C) STRANDEDNESS: single
(D) TOPOLOGY: linear

(ii) MOLECULE TYPE: cDNA to mRNA

(xi) SEQUENCE DESCRIPTION: SEQ ID NO: 2:

ATGGCGTCCG GCAGTAACTG GCTCTCCGGG GTGAATGTCG TGCTGGTGAT GGCCTACGGG 60

AGCCTGGTGT TTGTACTGCT ATTTATTTTT GTGAAGAGGC AAATCATGCG CTTTGCAATG 120

AAATCTCGAA GGGGACCTCA TGTCCCTGTG GGACACAATG CCCCCAAGGA CTTGAAAGAG 180

GAGATTGATA TTCGACTCTC CAGGGTTCAG GATATCAAGT ATGAGCCCCA GCTCCTTGCA 240

GATGATGATG CTAGACTACT ACAACTGGAA ACCCAGGGAA ATCAAAGTTG CTACAACTAT 300

CTGTATAGGA TGAAAGCTCT GGATGCCATT CGTACCTCTG AGATCCCATT TCATTCTGAA 360

GGCCGGCATC CCCGTTCCTT AATGGGCAAG AATTTCCGCT CCTACCTGCT GGATCTGCGA 420

AACACTAGTA CGCCTTTCAA GGGTGTACGC AAAGCACTCA TTGATACCCT TTTGGATGGC 480

TATGAAACAG CCCGCTATGG GACAGGGGTC TTTGGCCAGA ATGAGTACCT ACGCTATCAG 540

GAGGCCCTGA GTGAGCTGGC CACTGCGGTT AAAGCACGAA TTGGGAGCTC TCAGCGACAT 600

CACCAGTCAG CAGCCAAAGA CCTAACTCAG TCCCCTGAGG TCTCCCCAAC AACCATCCAG 660

GTGACATACC TCCCCTCCAG TCAGAAGAGT AAACGTGCCA AGCACTTCCT TGAATTGAAG 720

AGCTTTAAGG ATAACTATAA CACATTGGAG AGTACTCTG 759

(2) INFORMATION FOR SEQ ID NO: 3:

(i) SEQUENCE CHARACTERISTICS:
(A) LENGTH: 1628 base pairs
(B) TYPE: nucleic acid
(C) STRANDEDNESS: single
(D) TOPOLOGY: linear

(ii) MOLECULE TYPE: cDNA to mRNA

(xi) SEQUENCE DESCRIPTION: SEQ ID NO: 3:

GTCTGGCTTG GTCTTCCCCC GTAAGGAAAT GGCCGGGGAG CTCCAGGGGA CCCAGGCGCC 60

GTCGCTTCGG CGGAGCCTGG GCTGACCAGC CAGGACAGCG GGGTAAACCC GAACAATTCT 120

GCGCGAGGTA GGGAGGCCAT GGCGTCCGGC AGTAACTGGC TCTCCGGGGT GAATGTCGTG 180

CTGGTGATGG CCTACGGGAG CCTGGTGTTT GTACTGCTAT TTATTTTTGT GAAGAGGCAA 240

ATCATGCGCT TTGCAATGAA ATCTCGAAGG GGACCTCATG TCCCTGTGGG ACACAATGCC 300

CCCAAGGACT TGAAAGAGGA GATTGATATT CGACTCTCCA GGGTTCAGGA TATCAAGTAT 360

GAGCCCCAGC TCCTTGCAGA TGATGATGCT AGACTACTAC AACTGGAAAC CCAGGGAAAT 420

CAAAGTTGCT ACAACTATCT GTATAGGATG AAAGCTCTGG ATGCCATTCG TACCTCTGAG 480

ATCCCATTTC ATTCTGAAGG CCGGCATCCC CGTTCCTTAA TGGGCAAGAA TTTCCGCTCC 540

TACCTGCTGG ATCTGCGAAA CACTAGTACG CCTTTCAAGG GTGTACGCAA AGCACTCATT 600

GATACCCTTT TGGATGGCTA TGAAACAGCC CGCTATGGGA CAGGGGTCTT TGGCCAGAAT 660

GAGTACCTAC GCTATCAGGA GGCCCTGAGT GAGCTGGCCA CTGCGGTTAA AGCACGAATT 720

GGGAGCTCTC AGCGACATCA CCAGTCAGCA GCCAAAGACC TAACTCAGTC CCCTGAGGTC 780

TCCCCAACAA CCATCCAGGT GACATACCTC CCCTCCAGTC AGAAGAGTAA ACGTGCCAAG 840

CACTTCCTTG AATTGAAGAG CTTTAAGGAT AACTATAACA CATTGGAGAG TACTCTGTGA 900

CGGAGCTGAA GGACTCTTGC CGTAGATTAA GCCAGTCAGT TGCAATGTGC AAGACAGGCT 960

GCTTGCCGGG CCGCCCTCGG AACATCTGGC CCAGCAGGCC CAGACTGTAT CCATCCAAGT 1020

TCCCGTTGTA TCCAGAGTTC TTAGAGCTTG TGTCTAAAGG GTAATTCCCC AACCCTTCCT 1080

TATGAGCATT TTTAGAACAT TGGCTAAGAC TATTTTCCCC CAGTAGCGCT TTTTTCTGGA 1140

TTTGCATTCG GGTGTTATTC TTAATGTTTC TGTCAAAGCT TCTTAAAAAT CTTCACTTGG 1200

TTTCAGCCAT AGTTCACCTT CCCTGTTCCA GGTTTATTTA ATTCCAAAGG TGAGAGTTGG 1260

AGTGAGATGT CTTCCATATC TATACCTTTG TGCACAGTTG AATGGGAACT GTTTGGGTTT 1320

AGGGCATCTT AGAGTTGATT GATGGAAAAA GCAGACAGGA CTGGTGGGAG GTCAAGTGGG 1380

GAAGTTGGTG AATGTGGAAT AACTTACCTT TGTGCTCCAC TTAAACCAGA TGTGTTGCAG 1440

CTTTCCTGAC ATGCAAGGAT CTACTTTAAT TCCACACTCT CATTAATAAA TTGAATAAAA 1500

GGGAATGTTT TGGCACCTGA AATAATCTGC CAGGCTATGT GACAGTAGGN GGGAATGGTT 1560

TCCCCTNACA AGCCCAATGC ACTGGTCTGA CTTTATAAAT TATTTAATTA AATGAACTAT 1620

TATCAAAT 1628

(2) INFORMATION FOR SEQ ID NO: 4:

(i) SEQUENCE CHARACTERISTICS:
(A) LENGTH: 1628 base pairs
(B) TYPE: nucleic acid
(C) STRANDEDNESS: single
(D) TOPOLOGY: linear

(ii) MOLECULE TYPE: cDNA to mRNA

(vi) ORIGINAL SOURCE:
(A) ORGANISM: Homo sapiens
(H) CELL LINE: T98G

(ix) FEATURE:
(A) NAME/KEY: CDS
(B) LOCATION: 139..900

(xi) SEQUENCE DESCRIPTION: SEQ ID NO: 4:

GTCTGGCTTG GTCTTCCCCC GTAAGGAAAT GGCCGGGGAG CTCCAGGGGA CCCAGGCGCC 60

GTCGCTTCGG CGGAGCCTGG GCTGACCAGC CAGGACAGCG GGGTAAACCC GAACAATTCT 120

GCGCGAGGTA GGGAGGCC ATG GCG TCC GGC AGT AAC TGG CTC TCC GGG GTG 171
Met Ala Ser Gly Ser Asn Trp Leu Ser Gly Val
1               5                   10

AAT GTC GTG CTG GTG ATG GCC TAC GGG AGC CTG GTG TTT GTA CTG CTA 219

Asn Val Val Leu Val Met Ala Tyr Gly Ser Leu Val Phe Val Leu Leu
            15                  20                  25

TTT ATT TTT GTG AAG AGG CAA ATC ATG CGC TTT GCA ATG AAA TCT CGA 267
Phe Ile Phe Val Lys Arg Gln Ile Met Arg Phe Ala Met Lys Ser Arg
        30                  35                  40

AGG GGA CCT CAT GTC CCT GTG GGA CAC AAT GCC CCC AAG GAC TTG AAA 315
Arg Gly Pro His Val Pro Val Gly His Asn Ala Pro Lys Asp Leu Lys
    45                  50                  55

GAG GAG ATT GAT ATT CGA CTC TCC AGG GTT CAG GAT ATC AAG TAT GAG 363
Glu Glu Ile Asp Ile Arg Leu Ser Arg Val Gln Asp Ile Lys Tyr Glu
60                  65                  70                  75

CCC CAG CTC CTT GCA GAT GAT GAT GCT AGA CTA CTA CAA CTG GAA ACC 411
Pro Gln Leu Leu Ala Asp Asp Asp Ala Arg Leu Leu Gln Leu Glu Thr
                80                  85                  90

CAG GGA AAT CAA AGT TGC TAC AAC TAT CTG TAT AGG ATG AAA GCT CTG 459
Gln Gly Asn Gln Ser Cys Tyr Asn Tyr Leu Tyr Arg Met Lys Ala Leu
            95                  100                 105

GAT GCC ATT CGT ACC TCT GAG ATC CCA TTT CAT TCT GAA GGC CGG CAT 507
Asp Ala Ile Arg Thr Ser Glu Ile Pro Phe His Ser Glu Gly Arg His
        110                 115                 120

CCC CGT TCC TTA ATG GGC AAG AAT TTC CGC TCC TAC CTG CTG GAT CTG 555
Pro Arg Ser Leu Met Gly Lys Asn Phe Arg Ser Tyr Leu Leu Asp Leu
    125                 130                 135

CGA AAC ACT AGT ACG CCT TTC AAG GGT GTA CGC AAA GCA CTC ATT GAT 603
Arg Asn Thr Ser Thr Pro Phe Lys Gly Val Arg Lys Ala Leu Ile Asp
140                 145                 150                 155

ACC CTT TTG GAT GGC TAT GAA ACA GCC CGC TAT GGG ACA GGG GTC TTT 651
Thr Leu Leu Asp Gly Tyr Glu Thr Ala Arg Tyr Gly Thr Gly Val Phe
                160                 165                 170

GGC CAG AAT GAG TAC CTA CGC TAT CAG GAG GCC CTG AGT GAG CTG GCC 699
Gly Gln Asn Glu Tyr Leu Arg Tyr Gln Glu Ala Leu Ser Glu Leu Ala
            175                 180                 185

ACT GCG GTT AAA GCA CGA ATT GGG AGC TCT CAG CGA CAT CAC CAG TCA 747
Thr Ala Val Lys Ala Arg Ile Gly Ser Ser Gln Arg His His Gln Ser
        190                 195                 200

GCA GCC AAA GAC CTA ACT CAG TCC CCT GAG GTC TCC CCA ACA ACC ATC 795
Ala Ala Lys Asp Leu Thr Gln Ser Pro Glu Val Ser Pro Thr Thr Ile
    205                 210                 215

CAG GTG ACA TAC CTC CCC TCC AGT CAG AAG AGT AAA CGT GCC AAG CAC 843
Gln Val Thr Tyr Leu Pro Ser Ser Gln Lys Ser Lys Arg Ala Lys His
220                 225                 230                 235

TTC CTT GAA TTG AAG AGC TTT AAG GAT AAC TAT AAC ACA TTG GAG AGT 891

Phe Leu Glu Leu Lys Ser Phe Lys Asp Asn Tyr Asn Thr Leu Glu Ser
                240                 245                 250

ACT CTG TGACGGAGCT GAAGGACTCT TGCCGTAGAT TAAGCCAGTC AGTTGCAATG     947
Thr Leu

TGCAAGACAG GCTGCTTGCC GGGCCGCCCT CGGAACATCT GGCCCAGCAG GCCCAGACTG     1007

TATCCATCCA AGTTCCCGTT GTATCCAGAG TTCTTAGAGC TTGTGTCTAA AGGGTAATTC     1067

CCCAACCCTT CCTTATGAGC ATTTTTAGAA CATTGGCTAA GACTATTTTC CCCCAGTAGC     1127

GCTTTTTTCT GGATTTGCAT TCGGGTGTTA TTCTTAATGT TTCTGTCAAA GCTTCTTAAA     1187

AATCTTCACT TGGTTTCAGC CATAGTTCAC CTTCCCTGTT CCAGGTTTAT TTAATTCCAA     1247

AGGTGAGAGT TGGAGTGAGA TGTCTTCCAT ATCTATACCT TTGTGCACAG TTGAATGGGA     1307

ACTGTTTGGG TTTAGGGCAT CTTAGAGTTG ATTGATGGAA AAAGCAGACA GGACTGGTGG     1367

GAGGTCAAGT GGGGAAGTTG GTGAATGTGG AATAACTTAC CTTTGTGCTC CACTTAAACC     1427

AGATGTGTTG CAGCTTTCCT GACATGCAAG GATCTACTTT AATTCCACAC TCTCATTAAT     1487

AAATTGAATA AAAGGGAATG TTTTGGCACC TGAAATAATC TGCCAGGCTA TGTGACAGTA     1547

GGNGGGAATG GTTTCCCCTN ACAAGCCCAA TGCACTGGTC TGACTTTATA AATTATTTAA     1607

TTAAATGAAC TATTATCAAA T     1628

(2) INFORMATION FOR SEQ ID NO: 5:

(i) SEQUENCE CHARACTERISTICS:
(A) LENGTH: 41 base pairs
(B) TYPE: nucleic acid
(C) STRANDEDNESS: double
(D) TOPOLOGY: linear

(ii) MOLECULE TYPE: DNA (Synthetic DNA)

(xi) SEQUENCE DESCRIPTION: SEQ ID NO: 5:

CGTCGACAGA TCTGGTACCC GGGCGGCCGC ACTAGTCTGC A     41

(2) INFORMATION FOR SEQ ID NO: 6:

(i) SEQUENCE CHARACTERISTICS:
(A) LENGTH: 41 base pairs
(B) TYPE: nucleic acid
(C) STRANDEDNESS: double
(D) TOPOLOGY: linear

(ii) MOLECULE TYPE: DNA (Synthetic DNA)

(xi) SEQUENCE DESCRIPTION: SEQ ID NO: 6:

GACTAGTGCG GCCGCCCGGG TACCAGATCT GTCGACGAGC T 41

### Brief description of the drawings

Figure 1 shows the construction of plasmid vector, pVfCS-1.

Figure 2 shows the construction of a recombinant DNA into which cDNA derived from human glioblastoma cell line T98G is inserted.

## Claims

1. A polypeptide having the amino acid sequence shown in SEQ ID No. 1 in substantially purified form, a homologue thereof or a fragment of the sequence or homologue of a fragment.

2. A polypeptide according to claim 1 having the amino acid sequence shown in SEQ ID No. 1.

3. DNA encoding a polypeptide according to claim 1.

4. DNA according to claim 3 having the nucleotide sequence shown in SEQ ID No. 2 or a fragment thereof capable of selectively hybridizing to SEQ ID No. 2.

5. DNA according to claim 3 having the nucleotide sequence shown in SEQ ID No. 3 or a fragment thereof capable of selectively hybridizing to SEQ ID No. 3.

6. A replication and expression vector comprising DNA according to any one of claims 3 to 5.

7. Host cells transformed or tranfected with a replication and expression vector according to claim 6.

8. A method of producing a polypeptide which comprises culturing host cells according to claim 7 under conditions effective to express a polypeptide according to claim 1 or 2.

9. A monoclonal or polyclonal antibody to a polypeptide according to claim 1 or 2.

10. A pharmaceutical composition containing a polypeptide according to claims 1 or 2 or an antibody according to claim 9 in association with a pharmaceutically acceptable diluent and/or carrier.
